(19) Europäisches Patentamt
European Patent Office
Office européen des brevets

(11) Veröffentlichungsnummer : **0 093 976**
**B1**

(12) # EUROPÄISCHE PATENTSCHRIFT

(45) Veröffentlichungstag der Patentschrift : **10.09.86**

(21) Anmeldenummer : **83104196.7**

(22) Anmeldetag : **29.04.83**

(51) Int. Cl.⁴ : **C 07 C127/22**, C 07 C149/34, A 01 N 47/34, C 07 D319/20, C 07 C147/06, C 07 C143/02, C 07 D317/46, C 07 C149/32, C 07 D319/08// C07C157/12

(54) **2,4-Dihalogenbenzoyl-(thio)harnstoffe, Verfahren zu ihrer Herstellung und ihre Verwendung als Schädlingsbekämpfungsmittel.**

(30) Priorität : **11.05.82 DE 3217619**

(43) Veröffentlichungstag der Anmeldung : **16.11.83 Patentblatt 83/46**

(45) Bekanntmachung des Hinweises auf die Patenterteilung : **10.09.86 Patentblatt 86/37**

(84) Benannte Vertragsstaaten : **BE CH DE FR GB IT LI NL**

(56) Entgegenhaltungen :
**EP-A- 0 008 435**
**DE-A- 2 601 780**
**DE-A- 2 801 316**
**JOURNAL OF MEDICINAL CHEMISTRY, Band 12, 1969 M.H. ZAKARIA et al. "The bacteriostatic effectiveness of 1-acyl-3-(3,4-dichlorophenyl)ureas", Seiten 707-708**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Sirrenberg, Wilhelm, Dr.**
**Wuppertaler Strasse 21**
**D-4322 Sprockhoevel (DE)**
Erfinder : **Klauke, Erich, Dr.**
**Eichendorffweg 8**
**D-5068 Odenthal (DE)**
Erfinder : **Hammann, Ingeborg, Dr.**
**Lutherstrasse 22**
**D-4330 Mülheim/Ruhr (DE)**
Erfinder : **Stendel, Wilhelm, Dr.**
**In den Birken 55**
**D-5600 Wuppertal 1 (DE)**

**EP 0 093 976 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue 2,4-Dihalogenbenzoyl-(thio)harnstoffe, mehrere Verfahren zu ihrer Herstellung und ihre Verwendung als Schädlingsbekämpfungsmittel, insbesondere als Insektizide.

Es ist bereits bekannt, daß bestimmte Benzoylharnstoffe, wie z. B. 1-(2,6-Difluorbenzoyl)-3-(4-chlorphenyl)-harnstoff und 1-(2,6-Difluorbenzoyl)-3-(4-trifluormethylphenyl)-harnstoff, insektizide Eigenschaften besitzen (vgl. z. B. DE-AS 2 123 236 sowie die entsprechende US-PS 3 933 908 und US-PS 4 139 636, wobei die 2- und 2,6-substituierten Benzoylharnstoffe und -thioharnstoffe als besonders insektizid wirksam beschrieben wurden.

Es wurden die neuen 2,4-Dihalogenbenzoyl-(thio)harnstoffe der Formel I

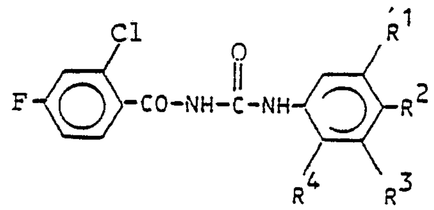

(I)

gefunden, in welcher

(1) $R^1$ und $R^3$ für Chlor,
$R^2$ für 4-Cyanophenoxy und
$R^4$ für Wasserstoff stehen ;
(2) $R^1$ und $R^3$ für Chlor,
$R^2$ für 2-Chlor-4-nitrophenoxy und
$R^4$ für Wasserstoff stehen ;
(3) $R^1$, $R^3$ und $R^4$ für Wasserstoff und
$R^2$ für 2-Chlor-4-trifluormethylphenoxy stehen ;
(4) $R^1$ für Chlor,
$R^2$ für 2-Chlor-4-cyanophenoxy und
$R^3$ und $R^4$ für Wasserstoff stehen ;
(5) $R^1$ und $R^4$ für Wasserstoff,
$R^2$ für 2,4-Dichlorphenoxy und
$R^3$ für Chlor stehen ;
(6) $R^1$, $R^3$ und $R^4$ für Wasserstoff und
$R^2$ für 2-Chlor-4-cyanophenoxy stehen ;
(7) $R^1$ und $R^3$ für Chlor,
$R^2$ für 4-Trifluormethoxyphenoxy und
$R^4$ für Wasserstoff stehen ;
(8) $R^1$ und $R^3$ für Chlor,
$R^2$ für 4-Trifluormethylthiophenoxy und
$R^4$ für Wasserstoff stehen und
(9) $R^1$ und $R^3$ für Chlor,
$R^2$ für 2-Chlor-4-trifluormethoxyphenoxy und
$R^4$ für Wasserstoff stehen.

Diese neuen Verbindungen weisen starke biologische, insbesondere insektizide Eigenschaften auf, die ihre Verwendung als Schädlingsbekämpfungsmittel, insbesondere als Insektizide ermöglichen.

Weiterhin wurde gefunden, daß die neuen 2,4-Dihalogenbenzoylharnstoffe der Formel (I) erhalten werden, indem man

a) substituierte Aniline der Formel II

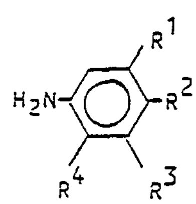

(II)

in welcher $R^1$, $R^2$, $R^3$ und $R^4$ die oben angegebenen Bedeutungen haben, mit dem Benzoyl-isocyanat der Formel III

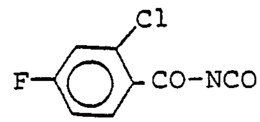

(III)

gegebenenfalls in Gegenwart eines Verdünnungsmittels umsetzt oder

b) substituierte Phenyl-isocyanate der Formel IV

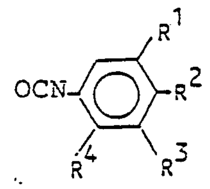

(IV)

in welcher $R^1$, $R^2$, $R^3$ und $R^4$ die oben angegebenen Bedeutungen haben, mit dem Benzoesäureamid der Formel V

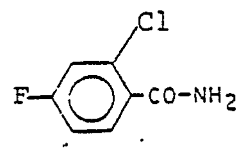

(V)

gegebenenfalls in Gegenwart eines Katalysators und gegebenenfalls in Gegenwart eines Verdünnungsmittels umsetzt.

Die als Ausgangsstoffe zu verwendenden substituierten Aniline der Formel (II) sind bekannt und nach literaturbekannten Verfahren und Methoden herstellbar (vgl. z. B. DE-OS 2 601 780, DE-OS 2 801 316, DE-OS 2 837 524, DE-OS 2 843 851 und DE-OS 3 023 328).

Die Ausgangsverbindung der Formel (III) ist bekannt.

Die Verbindungen der Formel (IV) sind bekannt oder können nach allgemein bekannten Verfahren und Methoden hergestellt werden.

Die Verbindung der Formel (V) ist bekannt.

Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone, wie Aceton, Methyl-ethyl-, Methyl-isopropyl- und Methylisobutylketon, Ester, wie Essigsäuremethylester und -ethylester, Nitrile, wie z. B. Acetonitril und Propionitril, Amide, wie z. B. Dimethylacetamid und N-Methylpyrrolidon, sowie Tetramethylensulfon.

Als Katalysatoren können für die Umsetzung gemäß Verfahrensvariante (b) vorzugsweise tertiäre Amine, wie Triethylamin und 1,4-Diazabicyclo [2,2,2]-octan, sowie organische Zinn-Verbindungen, wie z. B. Dibutylzinndilaurat verwendet werden.

Die Reaktionstemperatur kann innerhalb eines größeren Bereiches variiert werden. Im allgemeinen arbeitet man bei der Verfahrensvariante a) zwischen 20 und 180 °C, vorzugsweise zwischen 60 und 120 °C und bei der Verfahrensvariante b) zwischen 20 und 200°C, vorzugsweise zwischen 60 und 190 °C. Die erfindungsgemäßen Verfahrensvarianten werden im allgemeinen bei Normaldruck durchgeführt.

Zur Durchführung der erfindungsgemäßen Verfahrensvariante werden die Ausgangsstoffe gewöhnlich in etwa äquimolaren Mengen eingesetzt. Ein Überschuß der einen oder anderen Reaktionskomponente bringt keine wesentlichen Vorteile.

Die Aufarbeitung der Reaktionsprodukte geschieht nach üblichen Methoden, z. B. durch Absaugen des ausgefallenen Produktes oder durch Herauslösen von unerwünschten Nebenprodukten aus dem Reaktionsgemisch. Zur Charakterisierung dient der Schmelzpunkt.

Die Wirkstoffe eignen sich sehr gut zur Bekämpfung von tierischen Schädlingen, insbesondere Insekten, ganz besonders bevorzugt zur Bekämpfung von Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören :

Aus der Ordnung der Isopoda z. B. Oniscus asellus,
Armadillidium vulgare, Porcellio scaber.

Aus der Ordnung der Diplopoda z. B. Blaniulus guttulatus.

Aus der Ordnung der Chilopoda z. B. Geophilus carpophagus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp.

Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips femoralis, Thrips tabaci.

Aus der Ordnung der Heteroptera z. B. Eurygaster spp., Dysdercus intermedius, Piesma quadrata. Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z. B. Pectinophora gossypiella, Bupalus piniarius, Cheimatobia brumata, Lithocolletis blancardella, Hypnomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris ssp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aëdes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp.

Aus der Ordnung der Arachnida z. B. Scorpio maurus, Latrodectus mactans.

Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die neuen Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : stark polare Lösungsmittel, wie Dimethylformamid und Dimethylsulfoxid ; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid, als feste Trägerstoffe kommen in Frage : z. B. natürliche Gesteinsmehle, wie Kaoline, Tonerden, Talkum, Kreide, Quarz, Attpulgit, Montmorillonit oder Diatomeenerde und synthetische Gesteinsmehle, wie hochdisperse Kieselsäure, Aluminiumoxid und Silikate ; als

feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z. B. nichtionogene und anionische Emulgatoren, wie Polyoxyethylen-Fettsäure-Ester, Polyoxyethylen-Fettalkoholether, z. B. Alkylarylpolyglykolether, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Phenylharnstoffe, durch Mikroorganismen hergestellte Stoffe u. a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,000 000 1 bis zu 90 Gew.-% Wirkstoff, vorzugsweise zwischen 0,000 1 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten (mit Kalk gestrichenen) Unterlagen aus.

Die erfindungsgemäßen Wirkstoffe eignen sich auch zur Bekämpfung von Ekto- und Endoparasiten auf dem Gebiet der Tierhaltung und Viehzucht, wobei durch die Bekämpfung der Schädlinge bessere Ergebnisse, z. B. höhere Milchleistungen, höheres Gewicht, schöneres Tierfell, längere Lebensdauer usw. erreicht werden können.

Die Anwendung der erfindungsgemäßen Wirkstoffe geschieht auf diesen Gebieten in bekannter Weise, wie durch äußerliche Anwendung in Form beispielsweise des Tauchens (Dippen), Sprühens (Sprayen), Aufgießens (pour-on and spot-on) und des Einpuderns sowie durch orale Anwendung, beispielsweise über das Futter oder Trinkwasser in Form von beispielsweise Tabletten, Kapseln, Tränken, Granulaten.

Die Wirksamkeit der erfindungsgemäßen Stoffe sei anhand der folgenden Beispiele erläutert.

Auch der Formel (I) entsprechende Verbindungen mit Anilin-Resten, wie sie in den Verbindungen enthalten sind, welche in DE-AS 2 123 236 und 3 041 947 ; EP-OS 6 184, 7 687, 8 768, 13 414, 14 674, 14 675, 14 676, 16 729, 23 884, 25 363, 30 158, 31 974, 33 231, 35 084, 38 776, 40 179, 44 278 und 44 410 sowie JP-PS 55 011 537, 56 015 272 und 56 092 857 beschrieben werden, weisen als 2,4-Dihalogen-benzoyl-(thio)harnstoff-Derivate eine insektizide Wirksamkeit auf.

## Beispiel A

Plutella-Test

Lösungsmittel : 3 Gew.-Teile Dimethylformamid
Emulgator : 1 Gew.-Teil Alkylarylpolyglykolether

Zur Herstellung einer zweckmäßigen Wirkstoffzubereitung vermischt man 1 Gew.-Teil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden ; 0 % bedeutet, daß keine Raupen abgetötet wurden (Kontrolle).

Bei diesem Test zeigen z. B. bei einer Wirkstoffkonzentration von 0,001 % nach 7 Tagen beispielsweise die Verbindungen der Herstellungsbeispiele (30), (34), (40), (42) und (61) einen Abtötungsgrad von 100 %.

Die Herstellung der neuen Verbindungen soll anhand der folgenden Herstellungsbeispiele erläutert werden :

Beispiel 1

(Verfahrensvariante a)

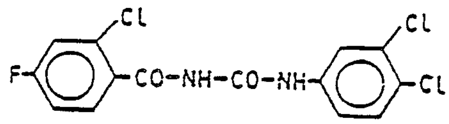

3,24 g (0,02 Mol) 3,4-Dichloranilin werden in 60 ml trockenem Toluol gelöst und unter Feuchtigkeitsausschluß mit 3,99 g (0,02 Mol) 2-Chlor-4-fluor-benzoyl-isocyanat versetzt. Der Ansatz wird eine Stunde bei 80 °C gerührt und dann auf 20-25 °C abgekühlt. Der ausgefallene Niederschlag wird abgesaugt und im Vakuum bei 100 °C getrocknet. Man erhält 6,8 g (94 % der Theorie) 1-(2-Chlor-4-fluor-benzoyl)-3-(3,4-dichlorphenyl)-harnstoff vom Schmelzpunkt 208 °C (nicht beanspruchte Verbindung).

Beispiel 2

(Verfahrensvariante b)

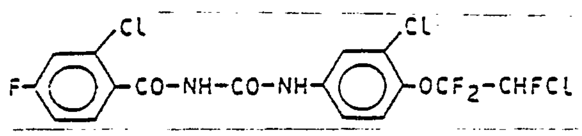

3,47 g (0,02 Mol) 2-Chlor-4-fluorbenzamid und 7,2 g (0,022 Mol) 3-Chlor-4-(2-chlor-1,1,2-trifluorethoxy)-phenyl-isocyanat werden in Gegenwart von einem Tropfen Dibutylzinndilaurat zwei Stunden bei 180 °C verschmolzen. Nach dem Abkühlen wird das Festprodukt in heißem Methanol zerkleinert, abgesaugt und getrocknet. Man erhält 7,2 g (76,5 % der Theorie) 1-(2-Chlor-4-fluor-benzoyl)-3-(3-chlor-4-(2-chlor-1,1,2-trifluor-ethoxy)-phenyl)-harnstoff vom Schmelzpunkt 186 °C (nicht beanspruchte Verbindung).

Analog den Beispielen 1 und 2 können die in der nachstehenden Tabelle aufgeführten erfindungsgemäßen Verbindungen der Formel (I) hergestellt werden.

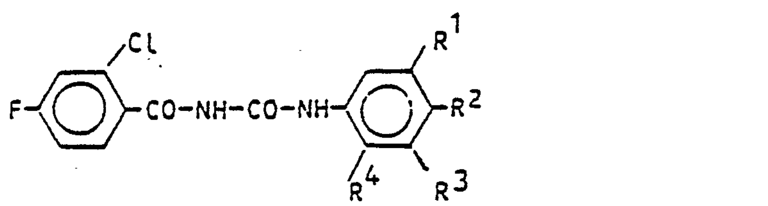

(I)

Tabelle

| Beisp. Nr. | $R^1$ | $R^2$ | $R^3$ | $R^4$ | Schmp. °C |
|---|---|---|---|---|---|
| 1 | Cl | 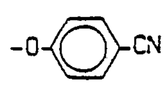 | Cl | H | 230 |
| 2 | Cl | 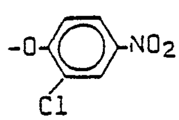 | Cl | H | 233 |
| 3 | H | 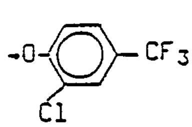 | H | H | 186 |

Tabelle (Fortsetzung)

| Beisp. Nr. | $R^1$ | $R^2$ | $R^3$ | $R^4$ | Schmp. °C |
|---|---|---|---|---|---|
| 4 | Cl | 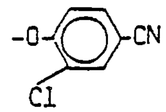 | H | H | 203 |
| 5 | H | 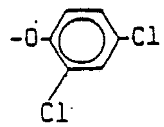 | Cl | H | 192 |
| 6 | H | 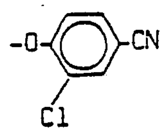 | H | H | 202 |
| 7 | Cl | 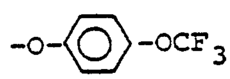 | Cl | H | 185 |
| 8 | Cl | 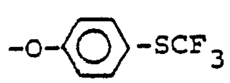 | Cl | H | 163 |
| 9 | Cl | 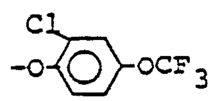 | Cl | H | 186 |

Die Herstellung der Ausgangsverbindung der Formel V (2-Chlor-4-fluorbenzamid) ist im folgenden beschrieben :

a) 573 g 2-Chlor-4-fluortoluol werden bei 100-140 °C unter UV-Bestrahlung bis zu einem Brechungsindex $n_D^{20}$ : 1,559 0 chloriert. Die Aufarbeitung durch Destillation ergibt :
978 g 2-chlor-4-fluor-benzotrichlorid vom
Kp. : 120 °C/20 mbar $n_D^{20}$ : 1,562 5.
b) 124 g des Chlor-fluorbenzotrichlorids werden vorgelegt auf 120 °C geheizt, 0,1 % $FeCl_3$ zugesetzt und dann tropfweise mit 9 g $H_2O$ versetzt. Es setzt eine zügige HCl-Entwicklung ein, die nach 1/2 h beendet ist. Aufarbeitung durch Destillation.
Ausbeute 65 g
Kp. 96 °C/15 mbar $n_D^{20}$ : 1,552 2.
c) Das Säurechlorid wird bei 30 °C in überschüssige ca. 12 % $NH_3$-Wasserlösung eingetropft und kräftig gerührt. Die abgeschiedenen Kristalle werden abfiltriert.

Aus 70 g Säurechlorid erhält man 60 g 2-Chlor-4-fluor-benzamid vom Fp : 149-51°.

**Patentansprüche**

1. 2,4-Dihalogenbenzoyl-(thio)harnstoffe der Formel (I)

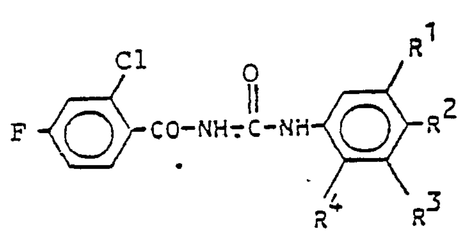
(I)

in welcher

(1) $R^1$ und $R^3$ für Chlor, $R^2$ für 4-Cyanophenoxy und $R^4$ für Wasserstoff stehen ;
(2) $R^1$ und $R^3$ für Chlor, $R^2$ für 2-Chlor-4-nitrophenoxy und $R^4$ für Wasserstoff stehen ;
(3) $R^1$, $R^3$ und $R^4$ für Wasserstoff und $R^2$ für 2-Chlor-4-trifluormethylphenoxy stehen ;
(4) $R^1$ für Chlor, $R^2$ für 2-Chlor-4-cyanophenoxy und $R^3$ und $R^4$ für Wasserstoff stehen ;
(5) $R^1$ und $R^4$ für Wasserstoff, $R^2$ für 2,4-Dichlorphenoxy und $R^3$ für Chlor stehen ;
(6) $R^1$, $R^3$ und $R^4$ für Wasserstoff und $R^2$ für 2-Chlor-4-cyanophenoxy stehen ;
(7) $R^1$ und $R^3$ für Chlor, $R^2$ für 4-Trifluormethoxyphenoxy und $R^4$ für Wasserstoff stehen ;
(8) $R^1$ und $R^3$ für Chlor, $R^2$ für 4-Trifluormethylthiophenoxy und $R^4$ für Wasserstoff stehen und
(9) $R^1$ und $R^3$ für Chlor, $R^2$ für 2-Chlor-4-trifluormethoxyphenoxy und $R^4$ für Wasserstoff stehen.

2. Verfahren zur Herstellung von 2,4-Dihalogenbenzoyl-(thio)harnstoffen der Formel (I)

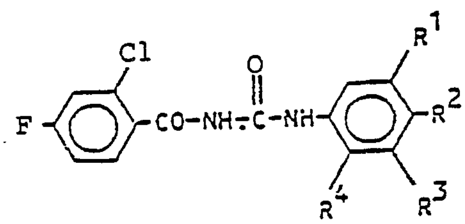

(I)

in welcher

(1) $R^1$ und $R^3$ für Chlor, $R^2$ für 4-Cyanophenoxy und $R^4$ für Wasserstoff stehen ;
(2) $R^1$ und $R^3$ für Chlor, $R^2$ für 2-Chlor-4-nitrophenoxy und $R^4$ für Wasserstoff stehen :
(3) $R^1$, $R^3$ und $R^4$ für Wasserstoff und $R^2$ für 2-Chlor-4-trifluormethylphenoxy stehen ;
(4) $R^1$ für Chlor, $R^2$ für 2-Chlor-4-cyanophenoxy und $R^3$ und $R^4$ für Wasserstoff stehen ;
(5) $R^1$ und $R^4$ für Wasserstoff, $R^2$ für 2,4-Dichlorphenoxy und $R^3$ für Chlor stehen ;
(6) $R^1$, $R^3$ und $R^4$ für Wasserstoff und $R^2$ für 2-Chlor-4-cyanophenoxy stehen ;
(7) $R^1$ und $R^3$ für Chlor, $R^2$ für 4-Trifluormethoxyphenoxy und $R^4$ für Wasserstoff stehen ;
(8) $R^1$ und $R^3$ für Chlor, $R^2$ für 4-Trifluormethylthiophenoxy und $R^4$ für Wasserstoff stehen und
(9) $R^1$ und $R^3$ für Chlor, $R^2$ für 2-Chlor-4-trifluormethoxyphenoxy und $R^4$ für Wasserstoff stehen,

dadurch gekennzeichnet, daß man

a) substituierte Aniline der Formel (II)

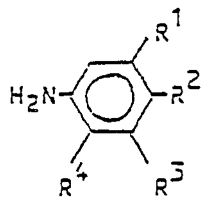

(II)

in welcher $R^1$, $R^2$, $R^3$ und $R^4$ die oben angegebenen Bedeutungen haben, mit dem Benzoyl-isocyanat der Formel (III)

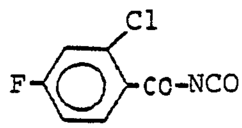

(III)

gegebenenfalls in Gegenwart eines Verdünnungsmittels umsetzt oder

b) substituierte Phenyl-isocyanate der Formel (IV)

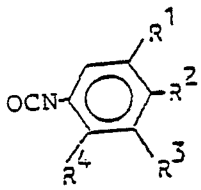

(IV)

in welcher $R^1$, $R^2$, $R^3$ und $R^4$ die oben angegebenen Bedeutungen haben, mit dem Benzoesäureamid der Formel (V)

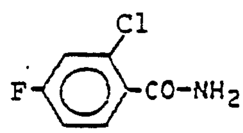

(V)

gegebenenfalls in Gegenwart eines Katalysators und gegebenenfalls in Gegenwart eines Verdünnungsmittels umsetzt.

3. Schädlingsbekämpfungsmittel, gekennzeichnet durch einen Gehalt an mindestens einer Verbindung der Formel (I).

4. Verwendung von Verbindungen der Formel (I) zur Bekämpfung von Schädlingen, insbesondere Insekten.

5. Verfahren zur Bekämpfung von Schädlingen, dadurch gekennzeichnet, daß man Verbindungen der Formel (I) auf die Schädlinge, vorzugsweise Insekten oder ihren Lebensraum einwirken läßt.

6. Verfahren zur Herstellung von Schädlingsbekämpfungsmitteln, dadurch gekennzeichnet, daß man Verbindungen der Formel (I) mit Streckmitteln und/oder oberflächenaktiven Mitteln vermischt.

**Claims**

1. 2,4-Dihalogenobenzoyl-(thio)ureas of the formula (I)

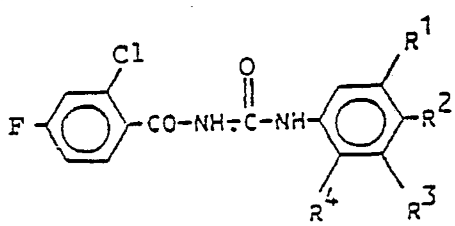

(I)

in which

(1) $R^1$ and $R^3$ represent chlorine,
$R^2$ represents 4-cyanophenoxy and

$R^4$ represents hydrogen ;
(2) $R^1$ and $R^3$ represent chlorine, $R^2$ represents 2-chloro-4-nitrophenoxy and $R^4$ represents hydrogen ;
(3) $R^1$, $R^3$ and $R^4$ represent hydrogen and $R^2$ represents 2-chloro-4-trifluoromethylphenoxy ;
(4) $R^1$ represents chlorine, $R^2$ represents 2-chloro-4-cyanophenoxy and $R^3$ and $R^4$ represent hydrogen ;
(5) $R^1$ and $R^4$ represent hydrogen, $R^2$ represents 2,4-dichlorophenoxy and $R^3$ represents chlorine ;
(6) $R^1$, $R^3$ and $R^4$ represent hydrogen and $R^2$ represents 2-chloro-4-cyanophenoxy ;
(7) $R^1$ and $R^3$ represent chlorine, $R^2$ represents 4-trifluoromethoxyphenoxy and $R^4$ represents hydrogen ;
(8) $R^1$ and $R^3$ represent chlorine, $R^2$ represents 4-trifluoromethylthiophenoxy and $R^4$ represents hydrogen and
(9) $R^1$ and $R^3$ represent chlorine, $R^2$ represents 2-chloro-4-trifluoromethoxyphenoxy and $R^4$ represents hydrogen.

2. Process for the preparation of 2,4-dihalogenobenzoyl-(thio)-ureas of the formula (I)

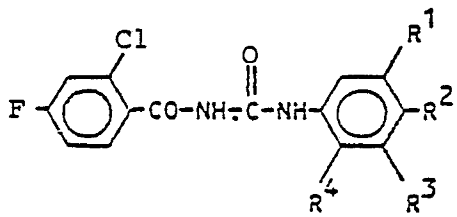

(I)

in which

(1) $R^1$ and $R^3$ represent chlorine, $R^2$ represents 4-cyanophenoxy and $R^4$ represents hydrogen ;
(2) $R^1$ and $R^3$ represent chlorine, $R^2$ represents 2-chloro-4-nitrophenoxy and $R^4$ represents hydrogen ;
(3) $R^1$, $R^3$ and $R^4$ represent hydrogen and $R^2$ represents 2-chloro-4-trifluoromethylphenoxy ;
(4) $R^1$ represents chlorine, $R^2$ represents 2-chloro-4-cyanophenoxy and $R^3$ and $R^4$ represent hydrogen ;
(5) $R^1$ and $R^4$ represent hydrogen, $R^2$ represents 2,4-dichlorophenoxy and $R^3$ represents chlorine ;
(6) $R^1$, $R^3$ and $R^4$ represent hydrogen and $R^2$ represents 2-chloro-4-cyanophenoxy ;
(7) $R^1$ and $R^3$ represent chlorine, $R^2$ represents 4-trifluoromethoxyphenoxy and $R^4$ represents hydrogen ;
(8) $R^1$ and $R^3$ represent chlorine, $R^2$ represents 4-trifluoromethylthiophenoxy and $R^4$ represents hydrogen and
(9) $R^1$ and. $R^3$ represent chlorine, $R^2$ represents 2-chloro-4-trifluoromethoxyphenoxy and $R^4$ represents hydrogen,

characterised in that
a) substituted anilines of the formula (II)

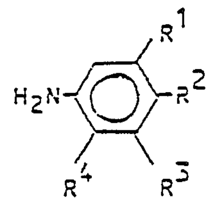

(II)

in which $R^1$, $R^2$, $R^3$ and $R^4$ have the abovementioned meanings, are reacted with the benzoyl isocyanate of the formula (III)

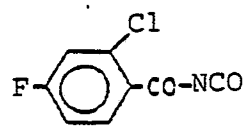

(III)

if appropriate in the presence of a diluent or

b) substituted phenyl isocyanates of the formula (IV)

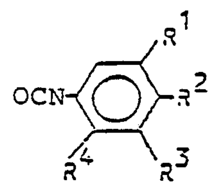

(IV)

in which $R^1$, $R^2$, $R^3$ and $R^4$ have the abovementioned meanings, are reacted with the benzoic acid amide of the formula (V)

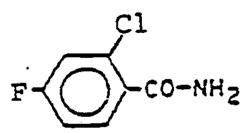

(V)

if appropriate in the presence of a catalyst and if appropriate in the presence of a diluent.

3. Agents for combating pests, characterised in that they contain at least one compound of the formula (I).

4. Use of compounds of the formula (I) for combating pests, in particular insects.

5. Method of combating pests, characterised in that compounds of the formula (I) are allowed to act on the pests, preferably insects, or their habitat.

6. Process for the preparation of agents for combating pests, characterised in that compounds of the formula (I) are mixed with extenders and/or surface-active agents.

**Revendications**

1. 2,4-dihalogénobenzoyl-(thio)urées de formule (I)

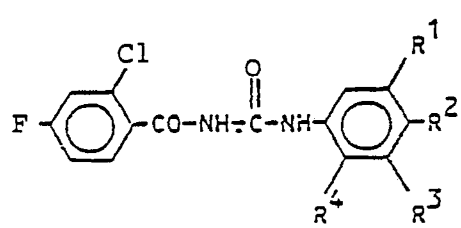

(I)

dans laquelle

(1) $R^1$ et $R^3$ représentent du chlore,
$R^2$ est un groupe 4-cyanophénoxy et
$R^4$ est de l'hydrogène ;

(2) $R^1$ et $R^3$ représentent du chlore,
$R^2$ est un groupe 2-chloro-4-nitrophénoxy et
$R^4$ est de l'hydrogène ;

(3) $R^1$, $R^3$ et $R^4$ sont de l'hydrogène et
$R^2$ est un groupe 2-chloro-4-trifluorométhylphénoxy ;
(4) $R^1$ représente le chlore,
$R^2$ est un groupe 2-chloro-4-cyanophénoxy et
$R^3$ et $R^4$ sont de l'hydrogène ;
(5) $R^1$ et $R^4$ sont de l'hydrogène,
$R^2$ est un groupe 2,4-dichlorophénoxy et
$R^3$ est du chlore ;
(6) $R^1$, $R^3$ et $R^4$ sont de l'hydrogène et
$R^2$ est le groupe 2-chloro-4-cyanophénoxy ;
(7) $R^1$ et $R^3$ représentent du chlore,
$R^2$ est un groupe 4-trifluorométhoxyphénoxy et
$R^4$ est de l'hydrogène ;
(8) $R^1$ et $R^3$ sont du chlore,
$R^2$ est un groupe 4-trifluorométhylthiophénoxy et
$R^4$ est de l'hydrogène ; et
(9) $R^1$ et $R^3$ représentent du chlore,
$R^2$ est un groupe 2-chloro-4-trifluorométhoxyphénoxy et
$R^4$ est de l'hydrogène.

2. Procédé de production de 2,4-dihalogénobenzoyl-(thio)urées de formule (I)

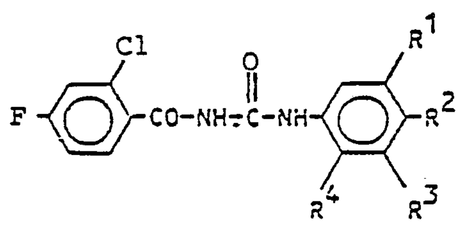

(I)

dans laquelle

(1) $R^1$ et $R^3$ représentent du chlore,
$R^2$ est un groupe 4-cyanophénoxy et
$R^4$ est de l'hydrogène ;
(2) $R^1$ et $R^3$ représentent du chlore,
$R^2$ est un groupe 2-chloro-4-nitrophénoxy et
$R^4$ est de l'hydrogène ;
(3) $R^1$, $R^3$ et $R^4$ sont de l'hydrogène et
$R^2$ est un groupe 2-chloro-4-trifluorométhylphénoxy ;
(4) $R^1$ représente le chlore,
$R^2$ est un groupe 2-chloro-4-cyanophénoxy et
$R^3$ et $R^4$ sont de l'hydrogène ;
(5) $R^1$ et $R^4$ sont de l'hydrogène,
$R^2$ est un groupe 2,4-dichlorophénoxy et
$R^3$ est du chlore ;
(6) $R^1$, $R^3$ et $R^4$ sont de l'hydrogène et
$R^2$ est le groupe 2-chloro-4-cyanophénoxy ;
(7) $R^1$ et $R^3$ représentent du chlore,
$R^2$ est un groupe 4-trifluorométhoxyphénoxy et
$R^4$ est de l'hydrogène ;
(8) $R^1$ et $R^3$ sont du chlore,
$R^2$ est un groupe 4-trifluorométhylthiophénoxy et
$R^4$ est de l'hydrogène ; et
(9) $R^1$ et $R^3$ représentent du chlore,
$R^2$ est un groupe 2-chloro-4-trifluorométhoxyphénoxy et
$R^4$ est de l'hydrogène,

caractérisé en ce que :
a) on fait réagir des anilines substituées de formule (II)

(Voir figure page 13)

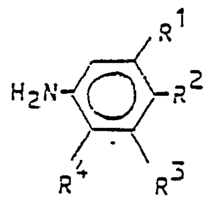

(II)

dans laquelle $R^1$, $R^2$, $R^3$ et $R^4$ ont les définitions indiquées ci-dessus, avec l'isocyanate de benzoyle de formule (III)

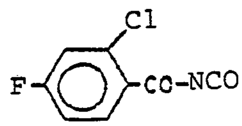

(III)

le cas échéant en présence d'un diluant, ou bien

b) on fait réagir des phénylisocyanates substitués de formule (IV)

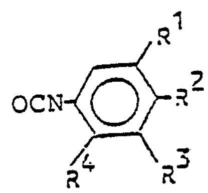

(IV)

dans laquelle $R^1$, $R^2$, $R^3$ et $R^4$ ont les définitions indiquées ci-dessus, avec l'amide d'acide benzoïque de formule (V)

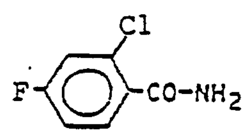

(V)

éventuellement en présence d'un catalyseur et, le cas échéant, en présence d'un diluant.

3. Compositions pesticides, caractérisées par une teneur en au moins un composé de formule (I).

4. Utilisation de composés de formule (I) pour combattre des parasites, en particulier des insectes.

5. Procédé pour combattre des parasites, caractérisé en ce qu'on fait agir des composés de formule (I) sur les parasites, de préférence des insectes, ou sur leur habitat.

6. Procédé de préparation de compositions pesticides, caractérisé en ce qu'on mélange des composés de formule (I) avec des diluants et/ou des agents tensio-actifs.